# EUROPÄISCHE PATENTANMELDUNG

(11) **EP 4 764 927 A1**
(43) Veröffentlichungstag der Anmeldung: **24.06.2026**
(21) Anmeldenummer: 24221126.6
(22) Anmeldetag: 18.12.2024
(51) Int. Cl.: G06F 21/62, G16H 10/60

(54) **VERFAHREN ZUR DATENTRANSKODIERUNG ZUR VERMEIDUNG DER RE-IDENTIFIKATION DURCH KI**

(71) Anmelder: Research Industrial Systems Engineering (RISE) Forschungs-, Entwicklungs- und Grossprojektberatung GmbH, 2320 Schwechat (AT)
(72) Erfinder: SCHÖNBAUER, Franz, 2320 Schwechat (AT); GRECHENIG, Thomas, 2320 Schwechat (AT); HÖLBLING, Dominik, 2320 Schwechat (AT)
(74) Vertreter: Schwarz & Partner Patentanwälte GmbH

(57) **Zusammenfassung**

Die Erfindung betrifft ein computerimplementiertes Verfahren zur Anonymisierung von personenbezogenen oder gesundheitsbezogenen Eingangsdatensätzen (DSj), umfassend die Schritte:
- Bereitstellen zumindest eines Eingangsdatensatzes (DSj), der Dateneinträge (DEi), insbesondere medizinische Messwerte, umfasst,
- Bestimmen einer positiven Obergrenze (pOG) und negativen Obergrenze (nOG) der Abweichung des zu transkodierenden Dateneintrags (DEi),
- Bestimmen einer positiven Untergrenze (pUG) und negativen Untergrenze (nUG) der Abweichung des zu transkodierenden Dateneintrags (DEi),
- wobei die positive Obergrenze (pOG) und die positive Untergrenze (pUG) einen oberen Wertebereich (oWB) bilden und die negative Obergrenze (nOG) und die negative Untergrenze (nUG) einen unteren Wertebereich (uWB) bilden;
- Zufälliges Wählen eines Zufallswertes im oberen oder unteren Wertebereich (oWB, uWB), um einen transkodierten Dateneintrag (tDEi) zu erhalten
- Ausgeben eines Ausgabedatensatzes, der den transkodierten Dateneintrag (tDEi) enthält.

## Beschreibung

Die Erfindung betrifft ein computerimplementiertes Verfahren zur Anonymisierung von personenbezogenen oder gesundheitsbezogenen Datensätzen.

Der Stand der Technik im Bereich der Anonymisierung personenbezogener und gesundheitsbezogener Daten hat sich in den letzten Jahren erheblich weiterentwickelt, da die Bedrohung durch Re-Identifikation, insbesondere durch den Einsatz künstlicher Intelligenz (KI), deutlich zugenommen hat. Traditionelle Anonymisierungstechniken wie die Pseudonymisierung oder die Aggregation von Daten mittels k-Anonymität weisen wesentliche Schwächen auf. Bei der Pseudonymisierung werden Identifikatoren durch Pseudonyme ersetzt, was jedoch keinen vollständigen Schutz bietet, da eine Re-Identifikation durch die Korrelation mit externen Datensätzen möglich ist. Die k-Anonymität, welche sicherstellt, dass eine Person nicht von mindestens k-1 anderen unterscheidbar ist, bietet einen guten Ansatz für die Anonymisierung, ist jedoch anfällig für fortschrittliche KI-Methoden, die versteckte Muster in den Daten erkennen können.

Die zunehmende Verfügbarkeit großer Datenmengen und die exponentiell steigende Rechenleistung von KI-Systemen ermöglichen es, auch stark anonymisierte Daten zu deanonymisieren. Künstliche Intelligenz kann dabei durch Cross-Referenzierung mit externen Datenbanken oder durch die Analyse von Verhaltensmustern in Datensätzen Rückschlüsse auf individuelle Personen ziehen. Traditionelle Techniken sind daher zunehmend unzureichend, um den Datenschutz in einer Welt zu gewährleisten, in der KI-basierte Analysen alltäglich geworden sind.

Vor diesem Hintergrund zeigt der Stand der Technik, dass Anonymisierungsmethoden weiter verbessert werden müssen. Moderne Ansätze bieten zwar verbesserten Schutz, stoßen jedoch bei der Verarbeitung großer und komplexer Datensätze an ihre Grenzen, insbesondere im Hinblick auf die Verhinderung der Re-Identifikation durch KI. Die fortschreitenden Entwicklungen erfordern daher robustere Lösungen, die diesen Herausforderungen begegnen können.

Es ist daher die Aufgabe der Erfindung, ein verbessertes Verfahren zur Anonymisierung von personenbezogenen oder gesundheitsbezogenen Datensätzen zu schaffen.

Diese Aufgabe wird gelöst durch ein computerimplementiertes Verfahren zur Anonymisierung von personenbezogenen oder gesundheitsbezogenen Datensätzen, umfassend die Schritte:
- Bereitstellen zumindest eines Eingangsdatensatzes, der Dateneinträge, insbesondere medizinische Messwerte, umfasst, wobei die Dateneinträge numerische Dateneinträge sind,
- Transkodieren zumindest eines Dateneintrags des Eingangsdatensatzes,
- Ausgeben eines Ausgabedatensatzes, der den transkodierten Dateneintrag umfasst, wobei das Transkodieren zumindest eines Dateneintrags durch die folgenden Schritte erfolgt:
- Bestimmen einer positiven und negativen Obergrenze der Abweichung des zu transkodierenden Dateneintrags, wobei die positive Obergrenze über dem Dateneintrag liegt und die negative Obergrenze unter dem Dateneintrag liegt,
- Bestimmen einer positiven und negativen Untergrenze der Abweichung des zu transkodierenden Dateneintrags, wobei die positive Untergrenze über dem Dateneintrag liegt und die negative Untergrenze unter dem Dateneintrag liegt,
- wobei die positive Obergrenze und die positive Untergrenze einen oberen Wertebereich bilden und die negative Obergrenze und die negative Untergrenze einen unteren Wertebereich bilden;
- Zufälliges Wählen eines Zufallswertes im oberen oder unteren Wertebereich, insbesondere durch Zufälliges Wählen des oberen oder unteren Wertebereichs und zufälliges Wählen eines Zufallswertes im zufällig gewählten Wertebereich, um den transkodierten Dateneintrag zu erhalten.

Erfindungsgemäß wird der Dateneintrag nicht nur z.B. gemäß einer Standardverteilung um den ursprünglichen Dateneintrag "verwischt", sondern der Dateneintrag wird zufällig in einem von zwei Wertebereichen gewählt, was die Re-Identifikation erheblich erschwert. Dabei ist festzuhalten, dass die Wertebereiche den ursprünglichen Dateneintrag dank der Untergrenze nicht umfassen und so einen gewissen Abstand zum ursprünglichen Dateneintrag erzwingen. Die Obergrenze ermöglicht dabei, dass der transkodierte Dateneintrag nicht zu weit vom ursprünglichen Dateneintrag weg liegt, was eine nachfolgende Auswertung des Ausgabedatensatzes verunmöglichen würde.

In anderen Worten dient die Festlegung der Untergrenze dazu, die Re-Identifikation so weit wie möglich zu verhindern, und die Obergrenze dient dazu, dass die nachfolgende Auswertung ermöglicht wird, jedoch auch, um eine Re-Identifikation zu erschweren, da diese umso schwerer wird, je weiter der transkodierte Dateneintrag vom ursprünglichen Dateneintrag weg liegt. Gleichzeitig ist ein möglichst großer Abstand zwischen positiver Obergrenze und positiver Untergrenze bzw. zwischen negativer Obergrenze und negativer Untergrenze vorteilhaft, um eine Re-Identifikation zu erschweren. Dies hat den Hintergrund, dass ein ursprünglicher Dateneintrag, der um eine konstante Zahl versetzt wurde, einfach auf den ursprünglichen Dateneintrag zurückrechenbar wäre.

Um dies nochmals zu verdeutlichen sei vorgebracht, dass die Obergrenze der Abweichung vom Ausgangswert durch einen disziplinspezifischen Maximalwert bestimmt werden kann, bei dem sichergestellt ist, dass sich eine abgeleitete Aussage durch die Änderung nicht verändert. z.B. Blutwertebereich, von dem kein anderes Krankheitsbild abgeleitet würde oder Finanzwerte innerhalb eines Investmentverlaufs, die keine andere Reichtums-Besitzkategorie oder Investmentstrategie bedeuten würden. Die Untergrenze der Abweichung vom Ausgangswert kann mittels einer Kombination aus statistischen Methoden berechnet werden, welche dazu dienen, die minimale Abweichung zu ermitteln, um auf Basis der Grundgesamtheit bzw. Wertemenge und der Wertevarianz sicherzustellen, dass mittels Machine Learning keine Re-Identifikation der Person stattfinden kann.

Besonders ist zudem, dass bei dem erfindungsgemäßen Verfahren die positive und negative Untergrenze nicht symmetrisch um den ursprünglichen Datensatz gewählt werden müssen, und auch die positive und negative Obergrenze müssen nicht symmetrisch um den ursprünglichen Datensatz gewählt. Dies kann insbesondere dann relevant sein, wenn eine Abweichung des ursprünglichen Datensatzes nach oben für eine weitere Auswertung unproblematisch ist, aber eine Abweichung des ursprünglichen Datensatzes nach unten eine weitere Auswertung erschweren würde, oder umgekehrt.

Es sei hervorgehoben, dass unter "Transkodieren" hierin insbesondere "nicht-rückführbares Transkodieren" oder "nicht-rückführbares Manipulieren" des Dateneintrags verstanden werden kann.

In einer besonders bevorzugten Ausführungsform der Erfindung umfasst das Verfahren die Schritte:
- Empfangen oder Bestimmen einer zu untersuchenden Eigenschaft, insbesondere eines Gesundheitszustandes wie einer Krankheit,
- Bestimmen der positiven und negativen Obergrenze in Abhängigkeit der zu untersuchenden Eigenschaft, insbesondere durch Abfragen einer Datenbank.

Dies ermöglicht, dass jeder Dateneintrag individuell transkodiert werden kann, und zwar in Abhängigkeit einer zu untersuchenden Eigenschaft. Um dies zu verstehen, wird folgendes fiktives Beispiel vorgebracht. Ein Dritter kann anfragen, dass er anonymisierte Datensätze benötigt, um Long-COVID zu untersuchen. In diesem Fall ist bekannt, dass für einen Dateneintrag einer bestimmten Kategorie, z.B. der Messwert "Ferritin" eines Blutbildes, eine überaus hohe Genauigkeit erforderlich ist, um Rückschlüsse auf Long-COVID zu ziehen. Die positive und negative Obergrenze werden entsprechend gering angegeben, z.B. als +/-0,1 % des Messwertes. Wenn der Dritte jedoch anfragt, dass er anonymisierte Datensätze benötigt, um einen bestimmten Typ von Krebs zu untersuchen, kann der Messwert "Ferritin" weiterhin relevant sein, aber weniger Einfluss auf die Auswertung haben, sodass eine positive und negative Obergrenze von z.B. +/-0,5 % des Messwertes herangezogen werden können, um die Auswertung nicht zu beeinflussen. Es versteht sich, dass bei einer Obergrenze von z.B. +/-0,5 % eine Re-Identifikation erheblich schwerer ist als bei einer Obergrenze von z.B. +/-0,1 %. Die individuellen Obergrenzen ermöglichen es somit, die Re-Identifikation je nach Abhängigkeit der zu untersuchenden Eigenschaft zu erschweren.

Weiters wird festgehalten, dass für bestimmte zu untersuchende Eigenschaften eine Abweichung nach oben oder bis zu einem bestimmten Schwellwert weniger oder mehr relevant sein kann als eine Abweichung nach unten oder bis zu einem bestimmten Schwellwert, sodass auch diese Asymmetrie von der zu untersuchenden Eigenschaft abhängen kann. Die positive und negative Obergrenze kann beispielsweise in einer Datenbank mit der zu untersuchenden Eigenschaft verknüpft sein, auf die während des Transkodierens zugegriffen wird. Im obigen Beispiel könnte die Datenbank beispielsweise einen ersten Dateneintrag "Long-COVID - Ferritin - 0,1%" und einen zweiten Dateneintrag "Krebs - Ferritin - 0,5%" umfassen. Alternativ könnte die positive und negative Obergrenze in der Anfrage eines Dritten unmittelbar angegeben werden.

Die positive und negative Untergrenze können im einfachsten Fall als Standardwert gewählt werden. Bevorzugt werden die positive und negative Untergrenze jedoch in Abhängigkeit von entsprechenden Dateneinträgen aus weiteren Eingangsdatensätzen oder Ausgangsdatensätzen gewählt, die einem Dritten übergeben werden sollen. Beispielsweise enthalten vier Datensätze jeweils einen Dateneintrag "Ferritin", d.h. einen Dateneintrag derselben Kategorie. Die Dateneinträge lauten jeweils 1.51, 1.49, 1.52 und 1.41. Würde man nun keine Untergrenze oder eine Untergrenze von +/-0.02 wählen, wäre es nicht möglich, den Dateneintrag des vierten Dateneinsatzes ausreichend zu anonymisieren, wodurch dieser Datensatz Re-identifizierbar wäre. Eine erfindungsgemäße Untergrenze von z.B. +/-0.08 hilft somit dabei, eine Re-Identifizierbarkeit erheblich zu erschweren. Für die ersten drei Datensätze könnte jedoch eine Untergrenze von +/-0.03 ausreichend sein. Es ist ersichtlich, dass die Untergrenze in Abhängigkeit von weiteren Dateneinträgen weiterer Datensätze gewählt werden sollte, um die Re-Identifizierbarkeit so weit wie möglich zu erschweren. In anderen Worten kann erfindungsgemäß zumindest ein weiterer Datensatz mit zumindest einem weiteren Dateneintrag bereitgestellt werden, der von der gleichen Kategorie wie der zu transkodierende Dateneintrag ist, wobei die positive und negative Untergrenze in Abhängigkeit des zumindest einen weiteren Dateneintrags ermittelt werden, insbesondere als zumindest die Hälfte des Abstandes zum nächstgelegenen weiteren Dateneintrags oder zumindest den Abstand zum nächstgelegenen weiteren Dateneintrag.

Insbesondere können die positive und negative Untergrenze in Abhängigkeit eines statistischen Verfahrens ermittelt werden, das bevorzugt aus einem der folgenden statistischen Verfahren gewählt ist:
- einer Berechnung der Intra- und Inter-Subjekt-Variabilität, insbesondere mittels ANOVA,
- einer Datenstandardisierung, insbesondre mittels z-Transformation,
- einer Clusteranalyse,
- einer Diskriminanzfunktionsanalyse, DFA,
- einer Definierung des Schwellenwertes mittels Receiver Operating Characteristics, ROC, Kurven,
- einer Validierung durch weitere Datensätze.

Die obigen Ausführungen zu den Obergrenzen und Untergrenzen sind in vielen Fällen ausreichend, um sowohl die Re-Identifikation zu erschweren als auch eine spätere Auswertung nicht zu beeinflussen. In manchen Fällen kommt es jedoch weiterhin dazu, dass eine Re-Identifikation möglich ist, und zwar wenn die Obergrenze und die Untergrenze zu nahe aneinander liegen. Es ist daher bevorzugt, wenn die positive und negative Untergrenze einen vorbestimmten Mindestabstand zur positiven und negativen Obergrenze einnehmen. Es liegen somit für jeden Wertebereich drei Freiheitsgrade vor, und zwar die Wahl der jeweiligen Untergrenze, die Wahl der jeweiligen Obergrenze und die Wahl des Abstandes zwischen Obergrenze und Untergrenze.

Im einfachsten Fall kann beim zufälligen Wählen eines Zufallswerts im zufällig gewählten Wertebereich keine Gewichtung vorgenommen werden, d.h. es ist genauso wahrscheinlich, dass der Zufallswert nahe an der Obergrenze oder nahe an der Untergrenze liegt. Um die spätere Auswertung jedoch möglichst wenig zu beeinflussen, kann bevorzugt werden, dass der Zufallswert beim zufälligen Wählen eines Zufallswerts im zufällig gewählten Wertebereich mit einer Gewichtung gewählt wird, wobei die Gewichtung bevorzugt an der positiven und/oder negativen Untergrenze den Maximalwert aufweist.

In einem weiteren Fall können der untere und obere Wertebereich symmetrisch sein, d.h. gleich groß und gleich weit vom ursprünglichen Dateneintrag beabstandet. In diesem Fall wird es keinen Unterschied machen, ob zuerst eine konkrete Auswahl zwischen unterem und oberem Wertebereich getroffen wird und erst danach ein Zufallswert in dem gewählten Wertebereich gewählt wird, oder ob beide Auswahlen gleichzeitig getroffen werden, d.h. eine einzige Zufallszahl gewählt wird, die gleichzeitig den Wertebereich und den Zufallswert im jeweiligen Werteberich angibt. Beispielsweise könnte eine Zufallszahl im Bereich zwischen 0 und 1 gewählt werden; wenn die Zufallszahl unter 0,5 liegt, wird der untere Wertebereich gewählt und wenn die Zufallszahl über 0,5 liegt, wird der obere Wertebereich gewählt. Die genaue Größe der Zufallszahl wird dann den Zufallswert angeben. Dies kann auch für allgemeinere Fälle umgesetzt werden, auch wenn der untere und obere Wertebereich nicht symmetrisch sind.

Ein unabhängiges Wählen von Wertebereichen und Zufallswert hat jedoch den Vorteil, dass eine größere Sicherheit gegen Deanonymisierung gegeben ist, da ein Zufallszahlengenerator jeweils zwei Zufallszahlenwerte mit unterschiedlichen Startwerten des Zufallszahlengenerators erzeugen kann, d.h. der Wertebereich wird durch den Zufallszahlengenerator mit einem ersten Startwert ermittelt und der Zufallswert wird durch den Zufallszahlengenerator mit einem zweiten Startwert ermittelt, was die Deanonymisierungssicherheit wesentlich erhöht. In anderen Worten ist bevorzugt, wenn das zufällige Wählen des oberen oder unteren Wertebereichs und das zufällige Wählen eines Zufallswertes im zufällig gewählten Wertebereich unabhängig voneinander stattfinden und jeweils mittels eines mit unterschiedlichen Startwerten eines Zufallszahlengenerators durchgeführt werden.

Bislang wurde stets davon ausgegangen, dass die Obergrenze und die Untergrenze im selben Wertebereich, d.h. Datenraum, wie der zu manipulierende Dateneintrag, d.h. der transkodierte Dateneintrag lag in derselben Größenordnung wie der ursprüngliche Dateneintrag. Das Verfahren kann jedoch auch den folgenden Schritt umfassen: Abbilden des transkodierten Dateneintrags, des zu transkodierenden Dateneintrags oder der Obergrenzen und der Untergrenzen auf einen neuen Wertebereich mittels einer vorgegebenen Funktion. Jede dieser drei Optionen wird zu dem Ergebnis führen, dass der transkodierte Dateneintrag in einem anderen Wertebereich, d.h. Datenraum, liegt als der ursprüngliche Dateneintrag. Zwar ist ein derartiges Abbilden des Dateneintrags auf einen neuen Wertebereich an sich umkehrbar, jedoch wird dadurch eine zusätzliche Stufe geschaffen, um die Interpretation des Ausgabedatensatzes zu erschweren. Beispielsweise können erste Blutmesswerte üblicherweise in einem Wertebereich von 1-10 liegen und zweite Blutmesswerte in einem Wertebereich von 100-1000. Wenn ein Dritter nun einen Dateneintrag von z.B. 200 sieht, ist eindeutig, dass dieser ein zweiter Blutmesswert sein muss, was die Auswertung des Datensatzes für den Dritten erleichtert. Wenn nun beide (oder einer) der Blutmesswerte auf einen Wertebereich von z.B. 2000-4000 abgebildet werden, ist aus einem Dateneintrag alleine nicht nachvollziehbar, ob es sich um einen ersten Blutmesswert oder einen zweiten Blutmesswert handelt. Überraschenderweise hat sich gezeigt, dass es auch bei den auf den neuen Wertebereich abgebildeten Messwerten möglich ist, eine Auswertung mittels KI bezüglich Krankheiten oder anderen zu untersuchenden Eigenschaft durchzuführen, da die relativen Eigenschaften der Messwerte auch im neuen Wertebereich erhalten bleiben. Beispielsweise wird ein ursprünglich hoher Cholesterinwert auch im neuen Wertebereich ein "hoher" Cholesterinwert bezüglich des neuen Wertebereichs bleiben. Gleichzeitig wurde durch das Abbilden auf den neuen Wertebereich jedoch die Anonymisierung weiter verbessert.

Es versteht sich, dass nicht nur ein Dateneintrag eines einzigen Eingangsdatensatzes transkodiert werden kann, sondern auch mehrere Dateneinträge eines Eingangsdatensatzes oder Dateneinträge mehrerer Eingangsdatensätze.

In anderen Worten kann das erfindungsgemäße Verfahren den Schritt des vollständigen Transkodierens eines Eingangsdatensatzes umfassen, wobei beim vollständigen Transkodieren des Eingangsdatensatzes alle numerischen Dateneinträge des Eingangsdatensatzes transkodiert werden; und/oder das Verfahren kann den Schritt des Transkodierens zumindest eines Dateneintrags einer Vielzahl von Eingangsdatensätzen umfassen, wobei das Verfahren bevorzugt weiters den Schritt des vollständigen Transkodierens einer Vielzahl von Eingangsdatensätzen umfassen kann, wobei die Ausgangsdatensätze bevorzugt in einer Datenbank gespeichert werden.

Das Verfahren kann auch weiterführende Schritte umfassen, wie z.B. den Schritt des Bereitstellens des Ausgabedatensatzes an einen Dritten und gegebenenfalls auch den Schritt des computerunterstützten Verarbeitens zumindest ein Teil des Ausgabedatensatzes durch den Dritten, wobei der Teil den transkodierten Dateneintrag umfasst. Dieser Teil kann gegebenenfalls als Trainingsdaten zum Trainieren eines KI-Modells verwendet werden.

Die Erfindung schafft weiters eine Vorrichtung zur Datenverarbeitung, umfassend Mittel zur Ausführung der Schritte des genannten Verfahrens. Weiters schafft die Erfindung ein Computerprogramm, umfassend Befehle, die bei der Ausführung des Programms durch einen Computer diesen veranlassen, die Schritte des genannten Verfahrens auszuführen, und einen computerlesbaren Datenträger, auf dem das genannte Computerprogramm gespeichert ist.

Um die vorliegende Erfindung besser zu veranschaulichen und deren Funktionsweise detailliert zu erklären, wird im Folgenden auf die beigefügte Figur Bezug genommen. Diese Abbildung dient der Verdeutlichung der technischen Merkmale der Erfindung. Die nachfolgende Beschreibung der Figur soll dazu beitragen, die strukturellen und funktionalen Eigenschaften der Erfindung eingehend zu verstehen und deren Vorteile und technischen Fortschritte gegenüber dem Stand der Technik zu erläutern. Darin zeigt:
Figur 1 zeigt mehrere personenbezogene Datensätze, die jeweils mehrere Dateneinträge umfassen.
Figur 2 zeigt das Transkodieren eines Dateneintrags in einem Datensatz.
Figur 3 zeigt das Transkodieren eines Dateneintrags anhand eines ersten Beispiels auf einem Zahlenstrahl.
Figur 4 zeigt das Transkodieren eines Dateneintrags anhand eines zweiten Beispiels auf einem Zahlenstrahl.

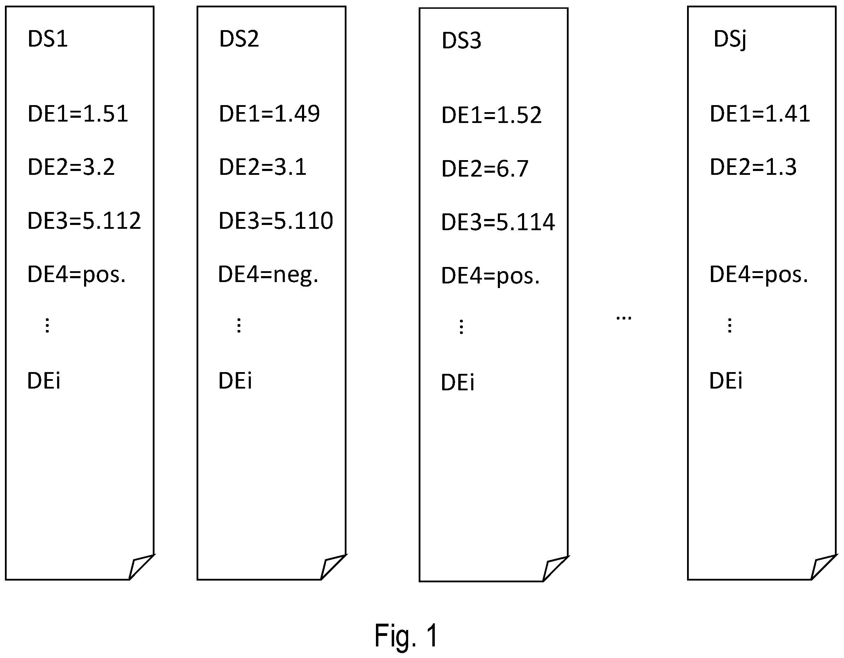

Gemäß Figur 1 geht die vorliegende Erfindung von zumindest einem Datensatz DE1 aus, in welchem personenbezogene Daten vorliegen. Personenbezogene Daten sind, z.B. nach der europäischen Datenschutz-Grundverordnung (DSGVO), alle Informationen, die sich auf eine identifizierte oder identifizierbare natürliche Person beziehen. Eine Person gilt als identifizierbar, wenn sie direkt oder indirekt identifiziert werden kann, etwa durch die Zuordnung zu einer Kennung wie einem Namen, einer Identifikationsnummer, Standortdaten oder einer Online-Kennung. Dazu gehören sowohl offensichtliche Informationen wie Name und Adresse, als auch weniger offensichtliche wie IP-Adressen oder GPS-Daten. Auch sensible Daten, etwa zu Gesundheit oder religiösen Überzeugungen, fallen unter den Begriff der personenbezogenen Daten, solange sie es ermöglichen, eine Person zu identifizieren. Beispielsweise kann aus der Kombination eines Geburtsdatums mit einer Adresse auf die Identität einer Person geschlossen werden.

Ein Sonderfall von personenbezogenen Daten sind gesundheitsbezogene Daten, die besonders schützenswert sind. Gesundheitsbezogene Daten sind, z.B. nach der europäischen Datenschutz-Grundverordnung (DSGVO), besondere Kategorien personenbezogener Daten, die Informationen über die körperliche oder geistige Gesundheit einer Person enthalten und aus denen Rückschlüsse auf den Gesundheitszustand dieser Person gezogen werden können. Dazu gehören Daten, die sich auf den gegenwärtigen, früheren oder zukünftigen Gesundheitszustand beziehen, wie Informationen über Erkrankungen, medizinische Behandlungen oder genetische und biometrische Daten, die zur eindeutigen Identifizierung einer Person dienen. Solche Daten unterliegen einem besonders hohen Schutz, da sie sensibel sind und einen tiefen Einblick in die Privatsphäre einer Person gewähren.

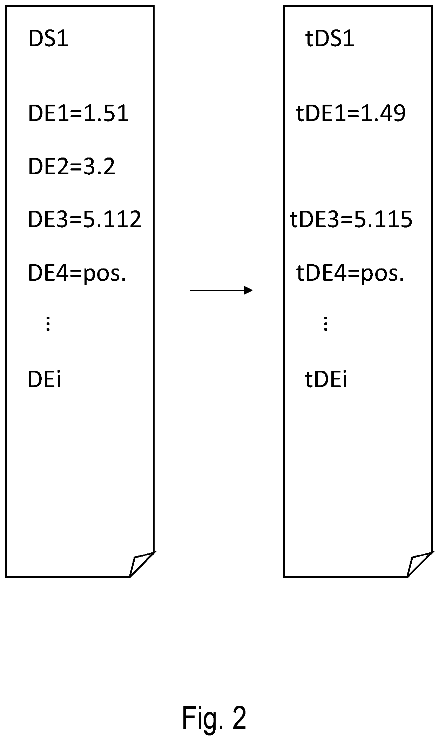

In Figur 1 ist ein erster Datensatz DS1 dargestellt, der mehrere Dateneinträge DE1, DE2, DE3, DE4, ... DEi aufweist. Dieser erste Datensatz DS1 umfasst "tatsächliche" Daten, z.B. medizinische Messdaten, oder das tatsächliche Geburtsdatum einer Person. Dieser Datensatz DSj kann auch als "Eingangsdatensatz" bezeichnet werden. Selbst wenn dieser Datensatz DSj keinen Namen oder dergleichen aufweist, kann dieser einer Person zugeordnet werden. Im obigen Beispiel der personenbezogenen Daten konnte durch die Kombination eines Geburtsdatums mit einer Adresse auf die Identität einer Person geschlossen werden. Aber auch wenn der Datensatz gesundheitsbezogene Daten umfasst und z.B. ein Blutbild ohne Namen ist, kann dieser einer Person zugewiesen werden, z.B. wenn ein Dritter denselben Datensatz mit Namen oder auch nur einen Teildatensatz des ersten Datensatzes mit Namen besitzt. Die unten im Zusammenhang mit den Figuren 2 bis 4 beschriebenen Verfahren, um den ersten Datensatz DSj zu anonymisieren, sodass dieser der Person nicht mehr zuordenbar ist, d.h. nicht mehr Re-Identifiziert werden kann.

Zuvor wird jedoch nochmals auf Figur 1 eingegangen, da aus dieser ersichtlich ist, dass mehrere Datensätze DS1, DS2, DS3, ... DSj, vorliegen können. Diese Datensätze DSj sind gleichfalls Eingangsdatensätze und umfassen "tatsächliche" Daten, z.B. medizinische Messdaten. Die Datensätze DSj stammen üblicherweise von unterschiedlichen Personen, können aber auch von derselben Person an unterschiedlichen Zeitpunkten aufgenommen worden sein. Diese Datensätze DSj können alle in einer Recheneinheit, wie einem sicherheitskritischen Rechenzentrum, vorliegen und dürfen von dieser nur anonymisiert ausgegeben werden. Es kann auch vorgesehen werden, dass die Eingangsdatensätze DSj sofort mit dem unten beschriebenen Verfahren anonymisiert werden, sobald sie vom Rechenzentrum empfangen werden, und nur in der anonymisierten Form gespeichert werden.

Die Datensätze DSj können alle eine gleiche Anzahl von Dateneinträgen DEi aufweisen, wobei dies jedoch nicht zwingend ist. In Figur 1 fehlt bei dem Datensatz DS3 beispielsweise der Dateneintrag DE3. Die Dateneinträge DEi, die bei der vorliegenden Erfindung transkodiert werden sollen, sind numerische Dateneinträge, insbesondere gebrochene Zahlen oder ganzzahlige Zahlen. Die numerischen Dateneinträge sind insbesondere medizinische Messwerte wie Blutmesswerte, Temperaturmesswerte, Blutdruckwerte, Pulsfrequenz, Atemfrequenz, Körpergewicht, Körpergröße oder Urinanalysen. In Figur 1 ist auch ersichtlich, dass die Datensätze DSj Dateneinträge DE4 umfassen können, die keine numerischen Dateneinträge sind. Diese werden nicht mit dem erfindungsgemäßen Verfahren transkodiert, können aber mit einem anderen Verfahren transkodiert werden.

Zur Definition wird weiters festgehalten, dass zwei Dateneinträge DEi von zwei oder mehreren Datensätzen DSj zur selben Kategorie gehören, wenn sie denselben Wert bezeichnen. Beispielsweise ist im Beispiel von Figur 1 der Dateneintrag DE1 der Blutmesswert "Ferritin", sodass der Dateneintrag DE1 des ersten Datensatzes DS1 und der Dateneintrag DE1 des zweiten Datensatzes DS2 derselben Kategorie angehören.

Unter einem anonymisierten Datensatz wird hierin ein Datensatz verstanden, bei dem zumindest einer der Dateneinträge DEi eines Eingangsdatensatzes DSj transkodiert wurde. Der Ausgabedatensatz, d.h. der anonymisierte Datensatz oder transkodierte Datensatz tDSj, kann beispielswiese alle Dateneinträge DEi (sowohl derselben Kategorie als auch mit dem selben inhaltlichen Zahlenwert) des Eingangsdatensatzes DSj umfassen, wobei jedoch zumindest einer der Dateneinträge DEi transkodiert wurde. Einige der Dateneinträge DEi können jedoch im Ausgabedatensatz tDSj auch weggelassen werden, insbesondere wenn diese nicht für eine weitere Auswertung benötigt werden, d.h. der Ausgabedatensatz tDSj kann gegenüber dem Eingangsdatensatz DSj sowohl transkodiert als auch reduziert sein. Wenn alle verbleibenden numerischen Dateneinträge tDEi im Ausgabedatensatz transkodiert sind, wird hierin auch von einem vollständig transkodierten Ausgabedatensatz tDSj gesprochen.

Figur 2 zeigt ein derartiges Beispiel, aus dem ersichtlich ist, dass aus einem Eingangsdatensatz DS1 ein Ausgabedatensatz tDS1 erzeugt wurde. Die numerischen Dateneinträge DE1, DE3 wurden transkodiert und werden als transkodierte Dateneinträge mDE1, mDE3 im Ausgabedatensatz tDS1 angeführt. Der Dateneintrag DE2 hat keinen Eingang in den Ausgabedatensatz DE2 gefunden. Der Dateneintrag DE4 ist kein numerischer Dateneintrag und wird für die vorliegende Erfindung nicht berücksichtigt.

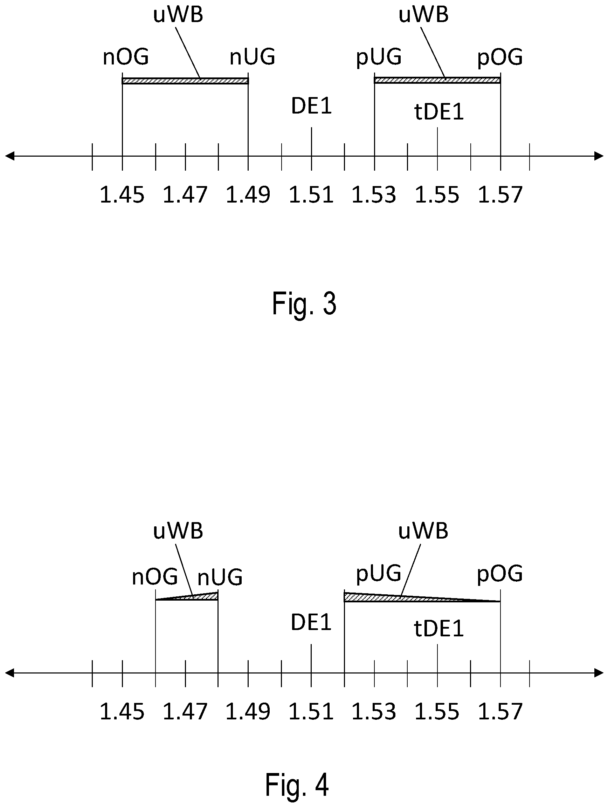

Figur 3 zeigt, wie die Dateneinträge DEi gemäß der vorliegenden Erfindung transkodiert werden. Der ursprüngliche Dateneintrag DE1 liegt gemäß diesem Beispiel bei dem Zahlenwert 1.51.

Zum Transkodieren wird eine positive Obergrenze pOG und eine negative Obergrenze nOG bestimmt. Die positive Obergrenze pOG und die negative Obergrenze nOG haben den Hintergrund, dass die ursprünglichen Dateneinträge DEi nicht über ein zu hohes Maß transkodiert werden dürfen, da dadurch eine spätere Auswertung nicht mehr möglich ist. Man kann sich vorstellen, dass es für die Bestimmung, ob eine bestimmte Krankheit vorliegt, keinen Unterschied macht, ob der Messwert 1.51 oder 1.55 beträgt. Würde der transkodierte Messwert jedoch 1.65 betragen, würde dies für die Bestimmung, ob eine bestimmte Krankheit vorliegt, einen wesentlichen Unterschied machen. Für eine andere Krankheit wäre es möglich, dass auch bei einem transkodierten Messwert von 1.65 kein Unterschied bei der Bestimmung einer Krankheit vorliegt, sondern erst bei einem transkodierten Messwert von 1.75.

Weiters wird zum Transkodieren eine positive Untergrenze pOG und eine negative Untergrenze nOG bestimmt. Die positive Untergrenze pOG und die negative Untergrenze nOG haben den Hintergrund, dass der transkodierte Dateneintrag tDEi möglichst wenig Rückschluss auf den ursprünglichen Dateneintrag DEi geben soll. Die Untergrenzen werden in der Regel in Abhängigkeit von Dateneinträgen DEi derselben Kategorie anderer Eingangsdatensätze DS bestimmt. Im dargestellten Beispiel sollen z.B. Untergrenzen für den Dateneintrag DE1=1.51 des ersten Datensatzes DS1 gefunden werden. Hierfür wird in den anderen Datensätzen DS2, DS3, DSj nach Dateneinträgen DE1 gesucht, welche derselben Kategorie wie der zu transkodierende Dateneintrag DE1 angehören, d.h. die Dateneinträge DE1=1.49, DE1=1.52 und DE1=1.41 (Figur 1). Alle vier Dateneinträge DE1 gehören derselben Kategorie an und sind z.B. "Ferritit"-Messwerte. Für den ersten Dateneintrag DE1 kann die positive Untergrenze z.B. bei 1.52 und die negative Untergrenze bei 1.49 gewählt werden, damit der transkodierte Dateneintrag DE1 im Bereich der anderen Dateneinträge DE1 liegt. Es sind jedoch auch andere statistische Verfahren möglich, um die Untergrenzen zu wählen, die auch nicht zwingend Dateneinträge anderer Dateneinsätze berücksichtigen müssen, insbesondere wenn statistische Schwankungsbreiten bereits vorbekannt sind.

Es versteht sich, dass die positive und negative Obergrenze pOG, nOG als auch die positive und negative Untergrenze pUG, nUG nicht mit dem ursprünglichen Dateneintrag DEi übereinstimmen. Die positive Obergrenze pOG weist zudem einen größeren Abstand zum ursprünglichen Dateneintrag DEi auf als die positive Untergrenze pUG. Die negative Obergrenze nOG weist einen größeren Abstand zum ursprünglichen Dateneintrag DEi auf als die negative Untergrenze nUG.

Sobald die positive und negative Obergrenze pOG, nOG als auch die positive und negative Untergrenze pUG, nUG bestimmt wurden, erhält man hieraus zwei Wertebereiche, und zwar einen oberen Wertebereich oWB zwischen der positiven Untergrenze pUG und der positiven Obergrenze pOG und einen unteren Wertebereich uWB zwischen der negativen Untergrenze nUG und der negativen Obergrenze nOG.

Um den transkodierten Dateneintrag tDEi zu bestimmen, wird eingangs ausgewählt, ob der obere Wertebereich oWB oder der untere Wertebereich uWB herangezogen werden soll. Im Beispiel von Figur 3 wurde der obere Wertebereich oWB herangezogen. Danach wird ein Zufallswert in dem zufällig ausgewählten Wertebereich oWB ausgewählt, der den transkodierten Dateneintrag tDEi darstellt. Diese beiden Schritte des Auswählens können unabhängig voneinander stattfinden oder gleichzeitig, z.B. indem die beiden Wertebereiche oWB, uWB fiktiv aneinandergehängt werden und eine Zufallszahl in diesem fiktiv zusammenhängenden Wertebereich gewählt wird.

Figur 4 zeigt ein weiteres Beispiel, bei dem der obere Wertebereich oWB oder der untere Wertebereich uWB asymmetrisch um den ursprünglichen Dateneintrag DE1 vorliegen. Dies Asymmetrie drückt sich hier in zwei Faktoren aus, und zwar haben die Wertebereiche oWB, uWB eine unterschiedliche Größe und zweitens sind diese unterschiedlich vom ursprünglichen Dateneintrag DE1 beabstandet. Es kann aber auch eine Asymmetrie vorgenommen werden, bei der nur einer dieser beiden Faktoren vorliegt.

In Figur 4 ist durch die dreieckigen, schraffierten Wertebereiche uWB, oWB weiters angedeutet, dass der Zufallswert mit einer Gewichtung gewählt wird und es wahrscheinlicher ist, dass dieser nahe an der positiven bzw. negativen Untergrenze pUG, nUG liegt als an der positiven bzw. negativen Obergrenze pOG, nOG. Im Beispiel von Figur 4 kommt eine lineare Gewichtung zum Einsatz, wobei sich versteht, dass auch andere Gewichtungen zum Einsatz kommen können, insbesondere Gewichtungen, die an eine Gaußkurve angelehnt sind, die in der Mitte durch den Abstand der positiven Untergrenze pUG von der negativen Untergrenze nUG getrennt ist. Zurückkommend auf Figur 3 ist ersichtlich, dass keine Gewichtung vorgenommen wurde und die Wahrscheinlichkeit des Zufallswertes im gesamten oberen und unteren Wertebereich uWB, oWB gleich ist.

Es sei nochmals hervorgehoben, dass alle hierin gezeigten Zahlenbeispiele nur dem Zwecke der Veranschaulichung von Spezialfällen dienen und insbesondere keine tatsächlichen Messdaten sind.

## Patentansprüche

1. Computerimplementiertes Verfahren zur Anonymisierung von personenbezogenen oder gesundheitsbezogenen Eingangsdatensätzen (DSj), umfassend die Schritte:
- Bereitstellen zumindest eines Eingangsdatensatzes (DSj), der Dateneinträge (DEi), insbesondere medizinische Messwerte, umfasst, wobei die Dateneinträge (DEi) numerische Dateneinträge sind,
- Transkodieren zumindest eines Dateneintrags (DEi) des Eingangsdatensatzes (DSj),
- Ausgeben eines Ausgabedatensatzes (tDSj), der den transkodierten Dateneintrag (tDEi) umfasst,
**dadurch gekennzeichnet, dass** das Transkodieren zumindest eines Dateneintrags (DEi) durch die folgenden Schritte erfolgt:
- Bestimmen einer positiven Obergrenze (pOG) und negativen Obergrenze (nOG) der Abweichung des zu transkodierenden Dateneintrags (DEi), wobei die positive Obergrenze (pOG) über dem Dateneintrag (DEi) liegt und die negative Obergrenze (nUP) unter dem Dateneintrag (DEi) liegt,
- Bestimmen einer positiven Untergrenze (pUG) und negativen Untergrenze (nUG) der Abweichung des zu transkodierenden Dateneintrags (DEi), wobei die positive Untergrenze (pUG) über dem Dateneintrag (DEi) liegt und die negative Untergrenze (nUG) unter dem Dateneintrag (DEi) liegt,
- wobei die positive Obergrenze (pOG) und die positive Untergrenze (pUG) einen oberen Wertebereich (oWB) bilden und die negative Obergrenze (nOG) und die negative Untergrenze (nUG) einen unteren Wertebereich (uWB) bilden;
- Zufälliges Wählen eines Zufallswertes im oberen oder unteren Wertebereich (oWB, uWB), um den transkodierten Dateneintrag (tDEi) zu erhalten.

2. Verfahren nach Anspruch 1, umfassend die Schritte:
- Empfangen oder Bestimmen einer zu untersuchenden Eigenschaft, insbesondere eines Gesundheitszustandes wie einer Krankheit,
- Bestimmen der positiven Obergrenze (pOG) und negativen Obergrenze (nOG) in Abhängigkeit der zu untersuchenden Eigenschaft, insbesondere durch Abfragen einer Datenbank.

3. Verfahren nach einem der vorhergehenden Ansprüche, wobei zumindest ein weiterer Datensatz (DSj) mit zumindest einem weiteren Dateneintrag (DEi) bereitgestellt wird, der von der gleichen Kategorie wie der zu transkodierende Dateneintrag (DEi) ist, wobei die positive Untergrenze (pUG) und negative Untergrenze (nUG) in Abhängigkeit des zumindest einen weiteren Dateneintrags (DEi) ermittelt werden, insbesondere als zumindest die Hälfte des Abstandes zum nächstgelegenen weiteren Dateneintrags (DEi).

4. Verfahren nach einem der vorhergehenden Ansprüche, wobei die positive Untergrenze (pUG) und negative Untergrenze (nUG) in Abhängigkeit eines statistischen Verfahren ermittelt werden, das bevorzugt aus einem der folgenden statistischen Verfahren gewählt ist:
- einer Berechnung der Intra- und Inter-Subjekt-Variabilität, insbesondere mittels Analysis of Variance, ANOVA,
- einer Datenstandardisierung, insbesondre mittels z-Transformation,
- einer Clusteranalyse,
- einer Diskriminanzfunktionsanalyse, DFA,
- einer Definierung des Schwellenwertes mittels Receiver Operating Characteristics, ROC, Kurven,
- einer Validierung durch weitere Datensätze.

5. Verfahren nach einem der vorhergehenden Ansprüche, wobei die positive Untergrenze (pUG) und negative Untergrenze (nUG) einen vorbestimmten Mindestabstand zur positiven Obergrenze (pOG) und negativen Obergrenze (nOG) einnehmen.

6. Verfahren nach einem der vorhergehenden Ansprüche, wobei der Zufallswert beim zufälligen Wählen mit einer Gewichtung gewählt wird, wobei die Gewichtung bevorzugt an der positiven und/oder negativen Untergrenze den Maximalwert aufweist.

7. Verfahren nach einem der vorhergehenden Ansprüche, umfassend den Schritt:
- Zufälliges Wählen des oberen Wertebereichs (oWB) oder unteren Wertebereichs (uWB) und zufälliges Wählen eines Zufallswertes im zufällig gewählten Wertebereich, um den transkodierten Dateneintrag (tDEi) zu erhalten,
wobei das zufällige Wählen des oberen Wertebereichs (oWB) oder unteren Wertebereichs (uWB) und das zufällige Wählen eines Zufallswertes im zufällig gewählten Wertebereich unabhängig voneinander stattfinden und jeweils mittels eines mit unterschiedlichen Startwerten eines Zufallszahlengenerators durchgeführt werden.

8. Verfahren nach einem der vorhergehenden Ansprüche, ferner umfassend den folgenden Schritt:
- Abbilden a) des transkodierten Dateneintrags (tDEi), b) des zu transkodierenden Dateneintrags (DEi) oder c) der positiven Obergrenze (pOG), negativen Obergrenze (nOG), positiven Untergrenze (pUG) und negativen Untergrenze (nUG) auf einen neuen Wertebereich mittels einer vorgegebenen Funktion.

9. Verfahren nach einem der vorhergehenden Ansprüche, umfassend den Schritt des vollständigen Transkodierens eines Eingangsdatensatzes (DSj), wobei beim vollständigen Transkodieren des Eingangsdatensatzes (DSj) alle numerischen Dateneinträge (DEi) des Eingangsdatensatzes (DSj) transkodiert werden.

10. Verfahren nach einem der vorhergehenden Ansprüche, umfassend den Schritt des Transkodierens zumindest eines Dateneintrags (DEi) einer Vielzahl von Eingangsdatensätzen (DSj), wobei das Verfahren bevorzugt weiters den Schritt des vollständigen Transkodierens einer Vielzahl von Eingangsdatensätzen (DSj), wobei die Ausgangsdatensätze (tDSj) bevorzugt in einer Datenbank gespeichert werden.

11. Verfahren nach einem der vorhergehenden Ansprüche, umfassend den Schritt des Bereitstellens des Ausgabedatensatzes (tDSj) an einen Dritten, bevorzugt weiters umfassend den Schritt des computerunterstützten Verarbeitens zumindest ein Teil des Ausgabedatensatzes (tDSj) durch den Dritten, wobei der Teil den transkodierten Dateneintrag (tDEi) umfasst.

12. Verfahren nach Anspruch 11 in Kombination mit Anspruch 2, umfassend den Schritt des computergestützten Untersuchens der Eigenschaft mittels des Ausgabedatensatzes (tDSj) durch den Dritten.

13. Vorrichtung zur Datenverarbeitung, umfassend Mittel zur Ausführung der Schritte des Verfahrens nach einem der Ansprüche 1 bis 10.

14. Computerprogramm, umfassend Befehle, die bei der Ausführung des Programms durch einen Computer diesen veranlassen, die Schritte des Verfahrens nach einem der Ansprüche 1 bis 10 auszuführen.

15. Computerlesbarer Datenträger, auf dem das Computerprogramm nach Anspruch 14 gespeichert ist.
